(19) **Europäisches Patentamt / European Patent Office / Office européen des brevets**

(11) **EP 4 316 531 A1**

(12) **EUROPEAN PATENT APPLICATION**
published in accordance with Art. 153(4) EPC

(43) Date of publication:
**07.02.2024 Bulletin 2024/06**

(21) Application number: **22727974.2**

(22) Date of filing: **29.03.2022**

(51) International Patent Classification (IPC):
**A61L 2/10** $^{(2006.01)}$          **A61L 9/20** $^{(2006.01)}$
**F21V 1/00** $^{(2006.01)}$

(52) Cooperative Patent Classification (CPC):
**A61L 9/20; A61L 2/10;** A61L 2202/14;
A61L 2202/25; A61L 2209/11

(86) International application number:
**PCT/IB2022/052896**

(87) International publication number:
**WO 2022/208352 (06.10.2022 Gazette 2022/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.03.2021 PT 2021117153**

(71) Applicant: **ALUVIA-ENGENHARIA E INVESTIGACAO, LDA.**
**4100 - 225 Porto (PT)**

(72) Inventors:
- **ROCHA AMARO NEVES, Carlos Manuel**
  **4400-478 VILA NOVA DE GAIA (PT)**
- **DE SOUSA CÁLIX, Antonio Paulo**
  **4500-263 ESPINHO (PT)**

(74) Representative: **Couto, Cláudia**
**Clarke, Modet Portugal**
**Av. Casal Ribeiro, 50 - 3º Dto.**
**1000-093 Lisboa (PT)**

(54) **MODELED UVC RAY EMITTER**

(57) The present invention describes a modeled UVC ray emitting apparatus (100) that automatically and sequentially irradiates different wavelengths, in the entire range of the UVC wavelength spectrum, namely between 200nm and 280nm, guaranteeing a significant increase on the sterilization effectiveness of areas where it is inserted.

The apparatus is composed of a stationary UVC ray emitting source of (1), which, through a set of tubes circumscribing said emitting source, and the fluid circulating inside said tubes, allows to promote the variation of said wavelength.

Fig. 1

## Description

## Technical field

The present invention describes a modeled UVC ray emitting apparatus.

## Background Art

Ultraviolet (UV) radiation is a well-known disinfectant for air, water and non-porous surfaces that has been effectively used for decades to reduce the spread of bacteria, organisms, viruses, fungi, etc.

The ability of this radiation in destroying this type of organisms is mostly within the range of the wavelength spectrum between 200nm and 280nm, that is, in the UVC range. For this reason, UVC lamps, often used for this purpose, are designated "germicidal" lamps.

However, and under the same conditions of intensity, exposure time and proximity to the emitting source, studies have shown that each pathogen agent responds differently and uniquely to the different wavelengths of said rays.

The equipment developed to date resorts to the use of a multiplicity of UV ray emitting sources with fixed wavelengths to guarantee a minimally effective result.

In the present approach, the aim is to overcome this state of the art limitation, guaranteeing the coverage of the entire UVC ray spectrum, focusing more effectively on the vulnerabilities of the type of agent to be targeted.

## Summary

The present application describes a modeled UVC ray emitting apparatus, characterized in that it comprises a support base; at least one stationary UVC ray emitting source adapted to an upper face of the support base in a central position; and a flow circuit adapted on the upper face of the support base, completely circumscribing the at least one stationary UVC ray emitting source in a central position; wherein the flow circuit models the wavelength emitted by the at least one stationary UVC ray emitting source.

In a proposed embodiment, the modeled UVC ray emitting apparatus comprises a thermal drive with flow variator.

In yet another embodiment, the flow circuit comprises a set of tubes made of a material with non-interfering characteristics in the radiation of the UVC spectrum.

In yet another embodiment, the set of tubes made of a material with non-interfering characteristics in the radiation of the UVC spectrum comprises within it the circulation of a fluid.

In yet another embodiment, the fluid is subject to temperature variation and pressure variation.

In yet another embodiment, the thermal drive with flow variator is adapted to promote the circulation of fluid within the flow circuit.

In yet another embodiment, the thermal drive with flow variator is adapted to promote the temperature variation of the fluid within the flow circuit.

In yet another embodiment, the thermal drive with flow variator comprises a pressure pump.

In yet another embodiment, the thermal drive with flow variator is adapted to promote the pressure variation of the fluid within the flow circuit.

In yet another embodiment, the temperature variation of the fluid within the flow circuit comprises values between 2 °C and 22 °C, and preferably between 4 °C and 20 °C.

In yet another embodiment, the fluid pressure variation within the flow circuit comprises values between 0.515568 kg/cm$^2$ and 1.000000 kg/cm$^2$.

In yet another embodiment, the flow circuit comprises a confluence ring adapted to ensure confluence of fluids within the flow circuit.

In yet another embodiment, the flow circuit comprises an expansion box, located in the confluence ring, responsible for ensuring the balance of the internal pressure in the circuit caused by the fluid circulating inside thereof, ensuring the accommodation of the fluid in expansion and compression moments.

In yet another embodiment, the flow circuit models the wavelength emitted by the at least one stationary UVC ray emitting source in a range between 200nm and 280nm.

The present application further describes the method of operation of the modeled UVC ray emitting apparatus according to the foregoing description comprising the steps of: 1. initializing the flow circuit; 2. After two minutes of the initialization process, the temperature of the fluid running through the flow circuit is raised and stabilized at 20 °C; 3. the emitting source lamp is activated; 4. After five minutes of emitting source lamp activation, the process of gradual cooling of the fluid running through the flow circuit begins over a period of five minutes until the temperature of the fluid running through the flow circuit stabilizes at 4 °C; 5. Once the temperature of the fluid running through the flow circuit has stabilized at 4 °C, the emitting source lamp is deactivated so that the pressure of the mercury vapor inside drops, the operating cycle returning to the step described in 2.

## Brief description

The present invention concerns an apparatus that automatically and sequentially irradiates different wavelengths, in the entire range of the UVC ray wavelength spectrum, namely between 200nm and 280nm.

The developed apparatus comprises the use of a fixed wavelength emitting source, allowing to guarantee the entire range of said spectrum between 200nm and 280nm through a modeling system of said UVC ray wavelength.

By using this apparatus, it is possible to guarantee the coverage of a wider spectrum, resulting, therefore, in a significant increase in the sterilization efficiency of the spaces. Thus, and with the approach of the developed system, each bacteriological and/or viral agent will be irradiated within the wavelength range for which it presents greater vulnerability, thus making it possible to increase the effectiveness of the elimination of these agents through a reduction of the exposure period required in about a third of the time when compared to existing systems, thus also increasing the universe of possible applications.

**Brief Description of the Figures**

For an easier understanding of the present application, figures are herein attached, which represent embodiments which however are not intended to limit the art herein disclosed.

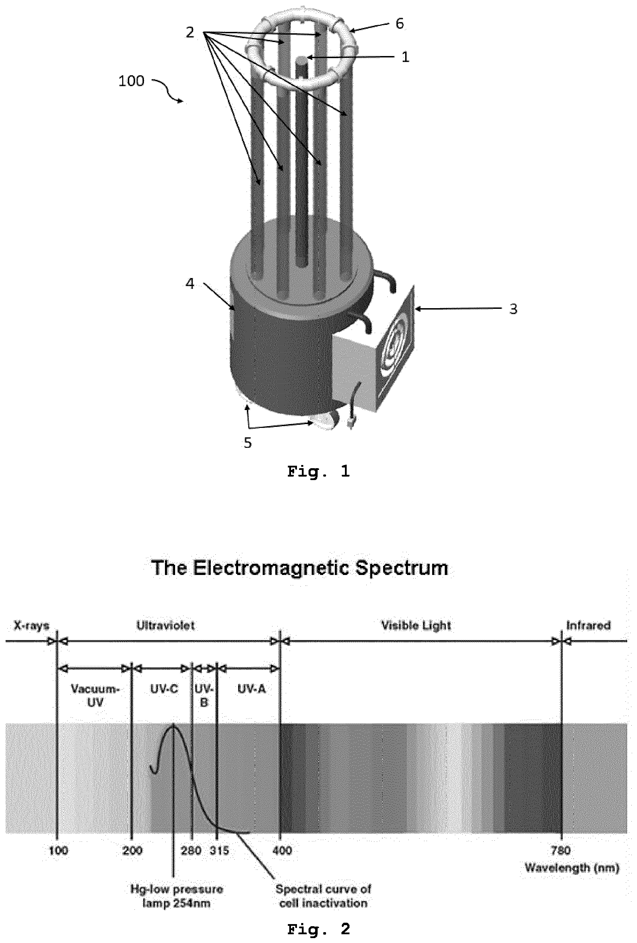

Figure 1 shows a view of the proposed apparatus. Reference numbers relate to:

    100. Modeled UVC ray emitting apparatus;
    1. stationary UVC ray source;
    2. flow circuit;
    3. thermal variator with flow control;
    4. support base;
    5. locomotion system;
    6. confluence ring.

Figure 2 shows the electromagnetic spectrum and the spectral curve of cell inactivation between UVC and UVB ray spectra, also referencing the wavelength of the stationary UVC ray emitting source (1) at 254nm.

**Description of the embodiments**

Referring to the figures, some embodiments are now described in more detail, which are not intended, however, to limit the scope of the present application.

The starting point for the development of the apparatus (100) is based on the knowledge that electromagnetic waves always move at a speed of 299.792 km/sec in vacuum, and when these waves cross different matter, their propagation speed is reduced. This phenomenon is related to the fact that light is dispersed among molecules making up the different media and does not affect photon speed. Thus, and for a given light frequency, the wavelength is proportional to wave speed,

$$c = v \times \lambda,$$

wherein c represents speed, v represents frequency and λ the wavelength. Therefore, for the case where a reduction in speed c is observed, the wavelength λ will also decrease, keeping the frequency v unchanged. Thus, the denser the medium to be traversed, the shorter the wavelength, and vice versa.

Thus, and based on this principle, the present application describes a modeled UVC ray emitting apparatus (100) comprising a stationary UVC ray emitting source (1), installed in a central position with respect to a flow circuit (2), or alternator system. The flow circuit (2) laterally circumscribes the stationary UVC source (1) and is responsible for modeling the wavelength emitted by the source (1), thus guaranteeing UVC ray emission covering the entire range of values between 200nm and 280nm.

The support base (4) of the modeled UVC ray emitting apparatus (100) comprises the use of a housing base for electrical and electronic circuits, being adapted to guarantee the safety and operation of the various modules of the emitter (100), and a cooling circuit arranged on the outside.

For one of the proposed embodiments, the UVC ray emitting source (1) of the apparatus (100) comprises a mercury vapor lamp, which at the beginning of its activity (start-up) emits a wavelength at 288nm, only reaching the peak of stable performance with a wavelength at 254nm after approximately five minutes, at which time there is sufficient vapor inside the lamp capable of generating the pressure necessary for the reaction. During the five heating minutes of the emitting source (1) lamp, and since during this heating the emitted rays cover wavelengths between 288nm and 254nm, said heating promotes by itself, the germicidal effect within such range. Once the stabilized/stationary operating point is reached, the remaining modeling of the rays emitted between 254nm and 200nm is therefore guaranteed by refraction through incidence thereof in the flow circuit (2).

The flow circuit (2), in one of the non-limiting proposed embodiments, comprises the use of a closed circuit of tubes, which in the perspective of increasing the versatility of the device (100), may comprise the use of multiple independent circuits. The flow circuit (2) comprises several transparent and/or translucent tubes arranged vertically, eight in one of the preferred non-limiting embodiments, which are interconnected by means of a closed confluence ring (6) which may be made of PVC or other material duly appropriate for the purpose. The vertical tubes of the flow circuit (2) have at least the same length as the stationary UVC ray emitting source (1) and are structurally constructed using a pure quartz composition so as not to interfere with the UVC spectrum emitted by the stationary source (1). The tubes of the flow circuit (2) comprise the use of a material with non-interfering characteristics in the radiation of the UVC spectrum, in one of the proposed embodiments in carbon-free pure quartz and approximately 2 mm thick, within which a fluid circulates, which, in the proposed embodiment, when subjected to thermal variations in a range between 2 °C and 22 °C, ideally between 4 °C and 20 °C, undergoes changes in density, respectively between $1000 g/cm^3$ and $0.998232$ $g/cm^3$, and pressure, respectively between $0.515568$ $kg/cm^2$ and $1.000000$ $kg/cm^2$. The circulating fluid may, in one of the several possible embodiments, comprise deionized and/or desalinated water to reduce the levels of sodium crystals, but alternatively other types of solutions may be used, namely electrically controlled variable density gel, gases, or others technically suitable for the purpose. The flow circuit (2) further comprises an expansion box, located in the confluence ring (6), responsible for ensuring the balance of the internal pressure in the circuit caused by the fluid circulating inside it, ensuring the accommodation of the expanded fluid at times of temperature rise.

The thermal variator with flow control (3) shall be responsible for the thermal control of the fluid inside the flow circuit tubes (2), as well as for controlling the flow and its circulation within. The thermal variator (3) comprises the use of a radiator to carry out the fluid cooling process, and a resistor to guarantee the heating thereof, a thermostat being used in both procedures to guarantee the accuracy of the temperature reading. The thermal variator (3) is pre-programmable to allow the programming of operating cycles according to the need, making it possible not only to define the temperature ranges, and corresponding range of UVC incidence, but also their action period. This programming allows adjusting the operation of the apparatus for specific functions, namely the elimination of specific viruses by exposure to particular wavelengths.

The thermal variator with flow control (3) also comprises a pressure pump that is responsible for controlling the pressure of the fluid present within the flow circuit (2), guaranteeing, together with the expansion box, the integrity and structural life span of the flow circuit tubes (2). Changing temperature, density and pressure of the fluid present inside the flow circuit (2) will promote the change in the wavelength of the rays emitted by the emitting source (1) when they cross the tubes. The forced circulation of the fluid inside the circuit tubes (2), as well as the control of temperature variations thereof, are ensured by a flow thermo-control device, designated thermal variator with flow control (3). Stabilizing the temperature at certain levels allows different values to be obtained along the aforementioned range, obtaining, for example, wavelengths between 235nm and 240nm. The versatility in obtaining different wavelengths makes it possible to define more clearly which virological and/or bacterial agents are to be eliminated. Thus, and with the possibility of varying the incident wavelengths, it is possible to optimize and improve the performance of the devices created for this purpose, since this variation of the emitted wavelength will allow to reduce the overdose of wavelengths, allowing a linear variation throughout the entire spectrum covered, and the reduction of exposure times in the surrounding application areas.

In a non-limiting and exemplifying way, the fluid present inside the flow circuit (2), at a temperature of 4 °C, promotes a deviation in the wavelength of the UVC emitted by the emitting source (1) to the 201nm range. For this temperature range, and in order to achieve the desired wavelength change, and using the pressure pump of the thermal variator with flow control (3), an approximate pressure of $0.515568$ kg/cm$^2$ is imposed on the fluid. Analogously, to obtain a deviation in the wavelength of the UVC emitted by the emitting source (1) to the 215nm range, the corresponding temperature of the fluid shall be approximately 7 °C and a pressure of $0.534748$ kg/cm$^2$. In order to obtain a deviation in the wavelength of the UVC emitted by the emitting source (1) for the 240nm range, the corresponding temperature of the fluid shall be approximately 12 °C and a pressure of $0.645718$ kg/cm$^2$.

The correlation between temperature, density and pressure of the fluid, associated with the variation and modification of the wavelength, is susceptible of non-limiting individual regulation for each type of application of the emitting apparatus (100). It should be noted that by way of example and for the particular case described, the proposed density for the fluid is $1.0000$ g/cm$^3$ or presents decimal and/or centesimal values very close thereto, presenting very slight variations.

The calibration and programming of the apparatus (100) regarding wavelengths emitted between 200nm and 280nm, can be guaranteed using a spectrometer, ensuring that a ten-minute operating cycle covers the entire aforementioned UVC spectrum.

The typology used also makes it possible to optimize and considerably lower the energy consumption of the apparatus (100), with its overall consumption being approximately 500 watts. In terms of dimensions, in one of the proposed embodiments, the modeled UVC ray emitting apparatus (100) will have a height of about 1.5 m and a width of 0.5 m. The stationary UVC ray emitting source (1), in one of the proposed non-limiting embodiments, comprises the use of a UVC ray emitting lamp with 350W power, with approximate dimensions of 1.20m x 20mm.

The method of operation of the modeled UVC ray emitting apparatus (100), in one of the proposed embodiments, comprises the steps of: 1. initializing the flow circuit (2); 2. after two minutes of the initialization process, the temperature of the fluid running through the flow circuit (2) is raised and stabilized at 20 °C; 3. the emitting source lamp (1) is activated; 4. After five minutes of emitting source lamp (1) activation, the process of gradual cooling of the fluid running through the flow circuit (2) begins over a period of five minutes until the temperature of the fluid running through the flow circuit (2) stabilizes at 4 °C; 5. Once the temperature of the fluid running through the flow circuit (2) has stabilized at 4 °C, the emitting source lamp (1) is deactivated so that the pressure of the mercury vapor inside drops, the operating cycle returning to the step described in 2.

The present description is of course in no way restricted to the embodiments presented herein and a person of ordinary skill in the art may provide many possibilities of modifying it without departing from the general idea as defined in the claims. The preferred embodiments described above are obviously combinable with each other. The following claims further define preferred embodiments.

**Claims**

1. Modeled UVC ray emitting apparatus (100), comprising:

   a support base (4);
   at least one stationary UVC ray emitting source (1) adapted to an upper face of the support base (4) in a central position; and
   a flow circuit (2) adapted on the upper face of the support base (4) completely circumscribing the at least one stationary UVC ray emitting source (1) in
   a central position;

   wherein flow circuit (2) models the wavelength emitted by the at least one stationary UVC ray emitting source (1) .

2. Modeled UVC ray emitting apparatus (100) according to the preceding claim, comprising a thermal drive with flow variator (3).

3. Modeled UVC ray emitting apparatus (100) according to the preceding claims, wherein the flow circuit (2) comprises a set of tubes made of a material with non-interfering characteristics in the radiation of the UVC spectrum.

4. Modeled UVC ray emitting apparatus (100) according to the preceding claims, wherein the set of tubes, made of a material with non-interfering characteristics in the radiation of the UVC spectrum, comprises within it the circulation of a fluid.

5. Modeled UVC ray emitting apparatus (100) according to the preceding claims, wherein the fluid is subject to temperature variation and pressure variation.

6. Modeled UVC ray emitting apparatus (100) according to the preceding claims, wherein the thermal drive with flow variator (3) is adapted to promote the circulation of fluid within the flow circuit (2).

7. Modeled UVC ray emitting apparatus (100) according to the preceding claims, wherein the thermal drive with flow variator (3) is adapted to promote the temperature variation of the fluid within the flow circuit (2).

8. Modeled UVC ray emitting apparatus (100) according to the preceding claims, wherein the thermal drive with flow variator (3) comprises a pressure pump.

9. Modeled UVC ray emitting apparatus (100) according to the preceding claims, wherein the thermal drive with flow variator (3) is adapted to promote the pressure variation of the fluid within the flow circuit (2).

10. Modeled UVC ray emitting apparatus (100) according to the preceding claims, wherein the temperature variation of the fluid within the flow circuit (2) comprises values between 2 °C and 22 °C, and preferably between 4 °C and 20 °C.

11. Modeled UVC ray emitting apparatus (100) according to the preceding claims, wherein the fluid pressure variation within the flow circuit (2) comprises values between 0.515568 kg/cm$^2$ and 1.000000 kg /cm$^2$.

12. Modeled UVC ray emitting apparatus (100) according to the preceding claims, wherein the flow circuit (2) comprises a confluence ring (6) adapted to ensure the confluence of fluids within the flow circuit (2).

13. Modeled UVC ray emitting apparatus (100) according to the preceding claims, wherein the flow circuit (2) comprises an expansion box, located in the confluence ring (6), responsible for ensuring the balance of internal pressure in the circuit caused by the fluid circulating inside thereof, ensuring the accommodation of the fluid in expansion and compression moments.

14. Modeled UVC ray emitting apparatus (100) according to the preceding claims, wherein the flow circuit (2) models the wavelength emitted by the at least one stationary UVC ray emitting source (1) in a range between 200nm and 280nm.

15. A method of operation of the modeled UVC ray emitting apparatus (100) according to claims 1 to 12 comprising the steps of:

   1. initializing the flow circuit (2);
   2. after two minutes of the initialization process, the temperature of the fluid running through the flow circuit (2) is raised and stabilized at 20 °C;
   3. the emitting source lamp (1) is activated;
   4. after five minutes of emitting source lamp (1) activation, the process of gradual cooling of the fluid running through the flow circuit (2) begins over a period of five minutes until the temperature of the fluid running through the flow circuit (2) stabilizes at 4 °C;
   5. Once the temperature of the fluid running through the flow circuit (2) has stabilized at 4 °C, the emitting source lamp (1) is deactivated so that the pressure of the mercury vapor inside drops, the operating cycle returning to the step

described in 2.

**Fig. 1**

## The Electromagnetic Spectrum

Hg-low pressure lamp 254nm

Spectral curve of cell inactivation

**Fig. 2**

# INTERNATIONAL SEARCH REPORT

| International application No |
|---|
| **PCT/IB2022/052896** |

**A. CLASSIFICATION OF SUBJECT MATTER**
INV. A61L2/10     A61L9/20     F21V1/00
ADD.

According to International Patent Classification (IPC) or to both national classification and IPC

**B. FIELDS SEARCHED**

Minimum documentation searched  (classification system followed by classification symbols)
A61L  F21V

Documentation searched other than minimum documentation to the extent that such documents are included  in the fields searched

Electronic data base consulted during the  international search (name of data base and,  where practicable, search terms used)

EPO-Internal

**C. DOCUMENTS CONSIDERED TO BE RELEVANT**

| Category* | Citation of document, with indication,  where appropriate, of the relevant passages | Relevant to claim No. |
|---|---|---|
| X | US 2017/128603 A1 (GUAMIS ALEGRE ALEXANDRE [ES] ET AL) 11 May 2017 (2017-05-11) paragraph: [0011], [0025], [0048]; figure: 1, 3 ----- | 1,2,5-15 |
| X | JP S63 133443 A (IWASAKI ELECTRIC CO LTD) 6 June 1988 (1988-06-06) page 1: line 27-29; page 2: line 1-12; fiugre: 3, 4 ----- | 1-6,8-15 |

☐ Further documents are listed in the  continuation of Box C.

☒ See patent family annex.

* Special categories of cited documents :

"A" document defining the general state of the art which is not considered to be of particular relevance

"E" earlier application or patent but published on or after the international filing date

"L" document which may throw doubts on priority  claim(s) or which is cited to establish the publication date of another  citation or other special reason (as specified)

"O" document referring to an oral disclosure, use,  exhibition or other means

"P" document published prior to the international filing date but later than the priority date claimed

"T" later document published after the international filing date or priority date and not in conflict with the application but cited to understand the principle or theory underlying the invention

"X" document of particular relevance;; the claimed invention cannot be considered novel or cannot be considered to involve an inventive step when the document is taken alone

"Y" document of particular relevance;; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| 8 July 2022 | 18/07/2022 |

| Name and mailing address of the ISA/ European Patent Office, P.B. 5818 Patentlaan 2 NL - 2280 HV Rijswijk Tel. (+31-70) 340-2040, Fax: (+31-70) 340-3016 | Authorized officer Nania, Manuela |

Form PCT/ISA/210 (second sheet) (April 2005)

## INTERNATIONAL SEARCH REPORT
Information on patent family members

| International application No |
| --- |
| **PCT/IB2022/052896** |

| Patent document cited in search report | | | Publication date | Patent family member(s) | | | Publication date |
| --- | --- | --- | --- | --- | --- | --- | --- |
| US 2017128603 | A1 | | 11-05-2017 | AU | 2015286615 | A1 | 02-02-2017 |
| | | | | BR | 112017000604 | A2 | 17-07-2018 |
| | | | | CA | 2954565 | A1 | 14-01-2016 |
| | | | | CN | 106573079 | A | 19-04-2017 |
| | | | | CO | 2017001312 | A2 | 30-06-2017 |
| | | | | DK | 2965766 | T3 | 24-07-2017 |
| | | | | EP | 2965766 | A1 | 13-01-2016 |
| | | | | ES | 2633114 | T3 | 19-09-2017 |
| | | | | HR | P20171047 | T1 | 06-10-2017 |
| | | | | HU | E035031 | T2 | 02-05-2018 |
| | | | | JP | 6588093 | B2 | 09-10-2019 |
| | | | | JP | 2017522159 | A | 10-08-2017 |
| | | | | KR | 20170030592 | A | 17-03-2017 |
| | | | | PL | 2965766 | T3 | 31-10-2017 |
| | | | | PT | 2965766 | T | 14-07-2017 |
| | | | | RU | 2662296 | C1 | 25-07-2018 |
| | | | | SI | 2965766 | T1 | 30-10-2017 |
| | | | | US | 2017128603 | A1 | 11-05-2017 |
| | | | | WO | 2016005556 | A1 | 14-01-2016 |
| JP S63133443 | A | | 06-06-1988 | NONE | | | |

Form PCT/ISA/210 (patent family annex) (April 2005)